Europäisches Patentamt

European Patent Office

Office européen des brevets

(19)

(11) Numéro de publication: **0 296 121**
**A2**

(12) ## DEMANDE DE BREVET EUROPEEN

(21) Numéro de dépôt: 88810402.3

(22) Date de dépôt: 14.06.88

(51) Int. Cl.⁴: **A 61 B 5/10**

(30) Priorité: 15.06.87 CH 2241/87

(43) Date de publication de la demande:
**21.12.88 Bulletin 88/51**

(84) Etats contractants désignés: **DE FR GB**

(71) Demandeur: **LIPATEC ETABLISSEMENT**
**Austrasse 27 Postfach 183**
**FL-9490 Vaduz (LI)**

(72) Inventeur: **Heinzer, Paul**
**Chemin des Oisillons 7**
**CH-1012 Pully/Lausanne (FR)**

(74) Mandataire: **Vimic, Milorad**
**Conseil en propriété industrielle La Prairie 3bis**
**CH-1400 Yverdon-les-Bains (CH)**

(54) **Distributeur individuel de liquide réactif pour la prise d'empreintes.**

(57) Le distributeur est formé d'un support imbibé de manière uniforme du liquide réactif et enfermé hermétiquement dans un sachet imperméable au liquide réactif et facile à ouvrir.

EP 0 296 121 A2

**Description**

## DISTRIBUTEUR INDIVIDUEL DE LIQUIDE REACTIF POUR LA PRISE D'EMPREINTES

La présente invention a pour objet un distributeur individuel de liquide réactif,pour la prise d'empreintes sur un support accepteur contenant au moins un coréactant pour une réaction colorée.

De longue date,une empreinte de la peau,par exemple l'empreinte d'un ou plusieurs doigts(empreinte digitale) ou d'une main(chiromancie)a été utilisée comme moyen sûr d'identification de personnes.L'extrémite antérieure des doigts est gravée d'une série de sillons séparant des crêtes de dessin complexe.Les empreintes présentent trois qualités qui leur permettent de jouer un très grand rôle dans l'identification d'un individu,à savoir l'immuabilité, l'inaltérabilité et la variété.L'empreinte se divise en régions centrale,basale et marginale et différentes méthodes de classification ont été mises au point.Une étude approfondie de cette science peut être trouvée dans l'ouvrage de Jean Gayet, intitulé "Manuel de police scientifique", édité par Payot,Paris.

La technique de prise d'empreintes est simple,mais il faut tenir compte d'un certain nombre de facteurs.En premier lieu,il faut utiliser une encre qui a des propriétés rhéologiques adaptées pour ce genre d'opération.L'encrage doit être exactement dosé;trop peu donne un dessin trop pâle où les crêtes et les sillons se différencient mal,un excès,au contraire,occasionne de larges maculations pri vées de tout détail.Pour ce faire,le doigt de la personne soumise à l'empreinte doit être "roulé" en une application franche sans aucun glissement,facteur de flou,et sans retour en arrière qui occasionne un guillochage des lignes et rend le tracé inexploitable.

L'utilisation d'encre noirede type offset permet la formation d'image permanente à haut contraste,mais sa mise en oeuvre est très désagréable.Pour obvier à cet inconvénient,on a proposé d'utiliser une réaction chimique colorée d'un réactif liquide,dont est imbibé un coussin, avec un coréactant contenu dans le support récepteur de l'empreinte.Citons,par exemple,la réaction d'un oxydant tel que le chlorure ferrique et de l'acide gallique(brevet US 2 082 735),de l'oxalate ferrique avec l'acide tannique(brevet US 2 104 586),du chlorure ferrique avec le 8-hydroxyquinoline(brevet US 4 262 623).D'autres réactions chimiques sont mentionnées dans les brevets US 2 235 632, US 4 029 012 et US 4 232 083.

L'importance de la quantité de liquide distribuée par le coussin encreur a été reconnue et bon nombre de systèmes ont été brevetés.Citons,par exemple,les brevets US 3 960 632, US 4 029 012, US 4 262 623 et US 4 379 178.Tous ses systèmes ont,sous une forme ou une autre,un coussin imbibé de liquide.De tels systèmes présentent l'inconvénient de se dessécher au cours du temps et,de ce fait,de ne pas distribuer une quantité uniforme de liquide.De plus,ces systèmes sont encombrants et,selon le type de construction, peuvent être d'un prix élevé.Un autre désavantage de tels systèmes est l'utilisation multiplé par différentes personnes de la même surface du coussin encreur.Ce fait est une barrière psychologique importante à l'utilisation plus étendue de la méthode de prise d'empreintes en vue d'identification de délinquants,d'enfants ou de nouveaux-nés, ou encore à l'établissements de diagnostiques médicaux.

L'appariton sur le marché d'appareils électroniques de lecture d'empreintes et de comparaison instantannée avec une empreinte de référénce,laisse entrevoir une utilisation plus large de cette technique d'identification (Fingermatrix Ridge Reader TM).

Le but de la présente invention est la réalisation d'un distributeur individuel de liquide réactif pour la prise d'empreintes pouvant être jeté après l'utilisation. Ce distributeur doit être d'une utilisation simple,bon marché et garder toute son efficacité au cours du temps de stockage qui peut être plus d'un an.

Un autre but de la présente invention est la réalisation d'un distributeur de dimension réduite,permettant la prise d'empreintes de grande surface,telles que les empreintes des mains,des oreilles,des pieds,des pneux ou des autres objets de grande surface.

A cet effet,le distributeur selon la présente invention est caractérisé par le fait qu'il est formé d'un support poreux imbibé de manière uniforme du liquide réactif et enfermé hermétiquement dans un sachet imperméable au liquide réactif et facile à ouvrir.

La présente invention est basée sur la découverte,au cours de la recherche de la solution au problème que pose la réalisation d'un distributeur simple et efficace de liquide réactif,qu'en imbibant de façon uniforme une serviette en papier adsorbant,pliée,d'une quantité contrôlée de liquide réactif,adaptée à sa porosité,on obtient un distributeur permettant de réaliser des images d'empreinte à haut pouvoir de résolution,la partie du pli de la serviette n'entraînant aucune discontinuité dans l'image de l'empreinte,ni de perte de contraste local.

Le support poreux peut être formé d'une feuille de fibres de cellulose ayant un bon pouvoir adsorbant pour des liquides.Des papiers de type crêpe ou buvard conviennent très bien.Le support poreux peut également être une feuille de fibres synthétiques,par exemple un papier syn thétique ou encore un support non tissé.D'autres supports adsorbants et de structure fine peuvent également convenir.

En ce qui concerne les constituants chimiques entrant dans la formation de l'imàge,un grand nombre de réactions connues dans le domaine de la formation d'images colorées peuvent être utilisées.Les brevets cités à titre de l'état de la technique donnent un apperçu des réactions déjà utilisées.D'autres réactifs possibles sont cités,par exemple, dans les brevets GB 1 298 194 et F 2 268 849,tout spécialement conçus pour des systèmes formateurs de colorants sensibles à la pression.

Le liquide utilisé pour imbiber le support poreux peut être aqueux ou organique.Il doit être adapté aux produits chimiques choisis pour la réaction coloré.Pour permettre une mise en solution du ou des réactants,on peut utiliser un mélange de liquides inertes et de solvants.Le paramètre de solubilité,la viscosité et la volatilité de chaque constituant liquide seront choisis pour permettre un bon mouillage du support poreux permettant une distribution uniforme du liquide à travers tout le support poreux plié.Si un solvant volatile est retenu dans la formulation du liquide réactif,sa quantité sera faible,afin d'éviter une variation de concentration de liquide réactif dans le support poreux en cours d'utilisation.Une certaine quantité de solvant dans le liquide réactif peut être benéfique pour acélérer la réaction chimique par solubilisition totale ou partielle du coréactant contenu dans le support accepteur sur lequel on forme l'image.Le support accepteur sur lequel est formée l'image de l'empreinte peut être une feuille de papier,une carte de sécurité ou tout autre support ou document.La composition chimique de sa surface comprendra le ou les coréactants du réactif mis en solution dans le liquide imbibant le support poreux.Il est important que le colorant une fois formé soit stable et ne migre pas au cours du temps.Ce support peut être pré imprimé avec des informations générales et des cases réservées aux empreintes,facilitant son utilisation.

La technologie de fabrication en grande série de sachets avec insertion de supports poreux pliés,injection contrôlée de liquide réactif et femeture hermétique de sachets est bien connue.L'application principale de cette technologie se situe dans la fabrication des serviettes rafraîchissantes.De tels sachets sont fabriqués,par exemple,parlvers Lee AG,CH-3400 Burgdorf et par Proderma AG, CH-6102 Malters.Des sachets contenant plusieurs serviettes sont également fabriqués pour le domaine d'application précité.

On donne ci-après les exemples de fabrication et d'utilisation de deux distributeurs selon la présente invention, pour la prise d'empreints palmaires et digitales,respectivement.

Exemple 1 - empreinte palmaire

On prépare la solution de liquide réactif par dissolution à 90°C des composants suivants:
100 g Meflex DC-29 (ICI)
5 g Pergascript I-2R (Ciba-Geigy)

Dans un sachet étanche,de dimension 65 x 80 mm,on introduit une feuille pliée de papier crêpe de 50 g/m$^2$(dimension de la feuille dépliée: 150 x 200 mm).On injecte par l'ouverture du sachet 1,2 g de la solution réactive sur le sommet de la feuille pliée et l'on ferme hermétiquement le sachet.Les entailles pratiquées de chaque côté du sachet,près de l'un de ses bords,permettent d'ouvrir facilement le sachet par déchirement.

Pour former une empreinte,on ouvre le sachet en le déchirant le long des entailles,on extrait la feuille pliée du sachet,on la déplie et on la pose à plat sur une surface plane.L'aspect de la feuille est très sec et sa manipulation ne cause pour ainsi dire aucune salissure des mains de l'opérateur.On appuie pendant une ou deux secondes,sans pression excessive,la main à dactyloscopier à plat sur la feuille,puis sur le support accepteur formé d'une feuille de papier de format A4 utilisée comme récepteur dans les sets de pap-vers sans carbone de type chimique (Papier Giroforum CF blanc 55 g/m$^2$ - Baumgartner Papier SA,CH-1023 Crissier),sans mouvement de rotation.On obtient immédiatement une image bleu,de haute intensité, de l'empreinte de la main.L'ensemble des lignes,crêtes papillaires et éminences sont redonnées avec une haute résolution.

L'image de l'empreinte ainsi obtenue est stable dans le temps.

Exemple 2 - empreinte digitale

On prépare la solution de liquide réactif par dissolution du colorant à chaud,puis,après refroidissement,on ajoute le solvant:
100 g Meflex DC-29 (ICI)
7 g Pergascript noir I-BR (Ciba-Geigy)
35 g Fréon TF (Du Pont de Nemours)

On procède ensuite comme dans l'exemple 1,pour obtenir un distributeur individuel conforme à la présente invention.

Il en est de même en ce qui concerne la formation d'une empreinte sauf en ce qui concerne le support accepteur qui est un support autocollant réactif de type MAC-ditto(Papierfabrik Biberist,CH-4562 Biberist)coupé aux dimensions 70 x 210 mm et préimprimé avec l'emplacement du nom,état civil et adresse de la personne à dactyloscopier et les cases pour les cinq doigts d'une main.

On obtient des empreintes noires à excellent pouvoir de résolution.

L'incompressibilité du support poreux imbibé du liquide réactif à la pression du doigt permet d'obtenir d'excellents résultats,quel que soit la manière dont l'empreinte est prise.La quantité de liquide restant sur le doigt après la prise d'empreinte est si minime qu'il n'est même pas nécessaire de se laver les mains.

L'empreinte obtenue est stable et se conserve très longtemps.

**Revendications**

1 Distributeur individuel du liquide réactif pour la prise d'empreintes sur un support accepteur contenant au moins un coréactant pour une réaction colorée,**caractérisé** par le fait qu'il est formé d'un support poreux imbibé de manière uniforme du liquide réactif et enfermé hermétiquement dans un sachet imperméable au liquide réactif et facile à ouvrir.

2. Distributeur selon la revendication 1,**caractérisé** par le fait que le support poreux est une feuille de fibres cellulosiques.

3. Distributeur selon la revendication 1,**caractérisé** par le fait que le support poreux est une feuille de fibres synthétiques.

4. Distributeur selon les revendications 1 à 3,**caractérisé** par le fait que le support poreux est plié dans le sachet.

5. Distributeur selon les revendications 1 à 4, **caractérisé** par le fait que le sachet est muni d'un système facilitant son ouverture par déchirement.

6. Distributeur selon la revendication 1, **caractérisé** par le fait que le liquide réactif comprend de l'eau.

7. Distributeur selon la revendication 1, **caractérisé**par le fait que le liquide réactif comprend au moins un liquide organique non volatil.

8. Distributeur selon les revendications 1 et 6, **caractérisé** par le fait que le liquide réactif comprend au moins un solvant organique.

9. Distributeur selon les revendications 1 et 7, **caractérisé** par le fait que le liquide réactif comprend au moins un solvant organique.

10. Utilisation du distributeur selon les revendications 1 à 9, **caractérisée** par le fait:

- que l'on sort du sachet le support poreux imbibé du liquide réactif;

- que l'on le déplie et pose à plat sur une surface plane;

- que l'on appuie la surface à dactyloscopier sur le support poreux imbibé du liquide réactif;

- que l'on place la surface ainsi humectée de liquide réactif sur un support contenant au moins un coréactant, pour former l'empreinte de la surface humectée par réaction colorée entre le réactif et le coréactant.